# EUROPÄISCHE PATENTSCHRIFT

(11) **EP 0 443 379 B1**
(45) Veröffentlichungstag und Bekanntmachung des Hinweises auf die Patenterteilung: **21.12.1994**
(21) Anmeldenummer: 91101726.7
(22) Anmeldetag: 08.02.1991
(51) Int. Cl.: A61B 17/22

(54) **Lithotripsie-Vorrichtung**
Lithotripsy installation
Appareil de lithotripsie

(30) Priorität: 20.02.1990 DE 4005228
(43) Veröffentlichungstag der Anmeldung: 28.08.1991
(73) Patentinhaber: Richard Wolf GmbH, 75438 Knittlingen (DE)
(72) Erfinder: Rentschler, Gunter, W-7527 Kraichtal-Münzesheim (DE); Burkhardt, Michael, W-7130 Mühlacker 7 (DE)
(74) Vertreter: Wilcken, Thomas, Dipl.-Ing.

(56) Entgegenhaltungen:
- EP-A- 0 265 741
- EP-A- 0 336 220

## Beschreibung

Die Erfindung betrifft eine Lithotripsie-Vorrichtung mit einem Stoßwellengenerator, umfassend ein an den Körper des Patienten anschmiegbares, Wasser als akustisches Koppelmedium zwischen dem Stoßwellengenerator und dem Körper des Patienten aufnehmendes Koppelkissen, eine Einrichtung zum Befüllen und Entleeren des Koppelkissens, eine Einrichtung zur Regelung des Flüssigkeitsdruckes in dem Koppelkissen und eine Einrichtung zum Entgasen des Koppelmediums.

Zum Eintragen von Ultraschall-Stoßwellen in die zu behandelnde Körperregion wird häufig Wasser als Koppelmedium benutzt, da es eine dem Körpergewebe von Lebewesen ähnliche akustische Impedanz aufweist. Dabei muß darauf geachtet werden, daß dieses Wasser möglichst gasfrei ist und daß während der Stoßwellentherapie entstehendes Gas aus dem Koppelmedium entfernt werden kann. Denn bei einem gashaltigen Koppelmedium ist aufgrund der veränderten akustischen Impedanz eine Stoßwellentherapie nicht mehr effizient durchzuführen. Gleichzeitig muß darauf geachtet werden, daß stets eine innige Verbindung zwischen Patient und Koppelmedium besteht, und zwar so, daß die Fokussierung der Stoßwellen auf das zu zerstörende Körperkonkrement stets exakt durchgeführt werden kann.

Eine Vorrichtung der einleitend angeführten Art ist in der DE-A-38 11 316 beschrieben. Bei dieser Vorrichtung wird ein geschlossenes Wasserkissen mit Wasser als Koppelmedium gefüllt und an dem Patienten angelegt, um die Stoßwellenenergie mittels des Wassers auf das zu zertrümmernde Konkrement des Patienten übertragen zu können. Mit einer Entgasungseinrichtung wird das Wasser des Wasserkissens entgast, und eine gesteuerte Druckregelungseinrichtung sorgt dafür, daß der Druck im Wasserkissen optimal ist. Jedoch kann sich das für das Kissen verwendete Wasser in seiner Qualität verschlechtern, z.B. durch Algenwachstum verschleimen, wodurch die Wirksamkeit der Stoßwellenenergieübertragung beeinträchtigt und damit der Behandlungserfolg verschlechtert wird. Ein Defekt im Wasserkreislauf für das Wasserkissen durch Austreten von Wasser ins Freie gefährdet des weiteren die Keimfreiheit da Raumes, in dem der Patient mit der Lithotripsie-Vorrichtung untersucht wird. Bei Verwendung eines offenen Wasserkissens besteht ebenfalls die Möglichkeit, daß das flüssige Koppelmedium in den Behandlungsraum unbeabsichtigterweise austreten kann und dann eine Säuberung und Desinfektion dieses Raumes oder Behandlungsplatzes erforderlich ist.

Bei einer bekannten Vorgehensweise zur berührungslosen Steinzertrümmerung gemäß der DE-A-35 44 628 und DE-A-32 20 751 erfolgt die Einkopplung der Ultraschall-Stoßwellen über einen mit entgastem Wasser gefüllten und gegen den Patienten offenen oder mit einer Membran gegen diesen abgeschlossenen und an den Körper des Patienten anschmiegbaren Koppelsack oder ein Koppelkissen. Als nachteilig erweist sich im Falle eines solchen abgeschlossenen Flüssigkeitskreislaufs, daß durch Algenwachstum und ähnliches schon nach kurzer Zeit eine Verschleimung des Koppelmediums auftreten kann. Ferner ist den beiden vorerwähnten Vorrichtungen nicht zu entnehmen, wie das als Koppelmedium dienende Wasser aufbereitet wird, um einer Verschleimung des Wassers vorzubeugen. Bezüglich der Entgasung ist lediglich erwähnt, daß im Bereich des Stoßwellengenerators und des Dichtrandes Entlüftungsschläuche eingeführt werden können.

Bei den Lithotriptoren der vorerwähnten Art besteht weiter eine Schwierigkeit, beim Belasten des Wasserkissens durch den Körper des Patienten den Anpreßdruck des Wasserkissens gegen den Körper auf dem optimalen Druckwert konstant zu halten. Denn bei der Positionierung des Patienten auf dem Koppelkissen übt das Koppelmedium einen Gegendruck zum Patienten aus, wodurch es möglich sein kann, daß der zu zertrümmernde Stein nicht in den Fokus der Stoßwellen zu bringen ist.

Aus der EP-A-02 65 741 ist eine Vorrichtung zum Zertrümmern von Konkrementen im Körper eines Lebewesens bekannt, die mit Flüssigkeit als Koppelmedium gefüllte Stoßwellengeneratoren und einen Flüssigkeitskreislauf mit Behälter und Umwälzpumpe aufweist. Der Flüssigkeitskreislauf ist unterteilbar in einen Betriebskreislauf und einen Füll- und Entgasungskreislauf. Im Füll- und Entgasungskreislauf wird die zugeführte Flüssigkeit mit Hilfe einer Vakuumpumpe entgast und auschließend in den bzw. die Stoßwellengeneratoren gefüllt. Die Flüssigkeit wird hierzu aus einem Vorratsbehälter entnommen.

Nachteilig bei dieser Vorrichtung ist daß aufgrund der zur Flüssigkeitsbevorratung und Flüssigeitsentgasung eingesetzten Behälter die gesamte Lithotripsievorrichtung viel Raum benötigt. Weiterhin verschleimt auch hier das Koppelmedium, so daß es innerhalb kurzer Zeit immer wieder ausgetauscht werden muß.

Neben den vorstehend angesprochenen Problemen ist insbesondere zu berücksichtigen, daß vor allem bei den patientenseitig nicht abgeschlossenen Koppelkissen eine Keimverschleppung über das Koppelmedium unter den Patienten erfolgen kann.

Die Aufgabe der Erfindung besteht in der Verbesserung einer Lithotripsie-Vorrichtung der einleitend angeführten Art dabingehend daß neben ihrem platzsparenden und konstuktiv einfachen Aufbau eine Sicherstellung der Qualität des Koppelmediums gewährleistet ist, wobei insbesondere als Koppelmedium Wasser aus dem üblichen Trinkwassernetz verwendbar ist.

Die Lösung dieser Aufgabe ist in dem Patentanspruch 1 angegeben, dessen Oberbegriff der oben erwähnten DE-A-38 11 316 entspricht. Mit dieser Lösung wird erreicht, daß durch Aufbereitung die Qualität des direkt aus dem Trinkwassernetz entnehmbaren Koppelmediums erhalten bleibt oder sogar verbessert wird, denn das Koppelmedium wird desinfiziert, so daß sich darin keine Keime bilden können oder kein Algenwachstum entstehen kann. Eine Verschleimung des Koppelmediums ist somit vermeiden, wodurch die Übertragungsqualität der Ultraschall-Stoßwellenenergie auf den Körper des Patienten gewährleistet ist und auf einem hohen Niveau stattfindet. Desgleichen ist es bei einem Defekt der Lithotripsie-Vorrichtung oder bei Verwendung eines offenen Koppelkissens nicht mehr möglich, daß die Keimfreiheit des Arbeitsplatzes bzw. des Behandlungsraumes gefährdet ist und ein Desinfizieren desselben entfällt. Das Vorsehen der erfindungsgemäßen Desinfektionseinrichtung vergrößert nur unwesentlich die Lithotripsie-Vorrichtung, so daß deren platzsparender und einfacher Aufbau möglich ist bzw. erhalten bleibt.

Vorzugsweise Ausgestaltungen der erfindungsgemäßen Vorrichtung sind in den Unteransprüchen angeführt. So ist es bei kleineren Dosiermengen an Desinfektionsmittel vorteilhaft, daß die Dosierpumpe der Desinfektionseinrichtung intermittierend angesteuert wird. Des weiteren kann zwecks Erzielung einer lagen Gebrauchsdauer des aufbereiteten Koppelmediums eine Rohrleitung vorgesehen sein, die den Hauptkreislauf der Vorrichtung mit der Desinfektionseinrichtung verbindet.

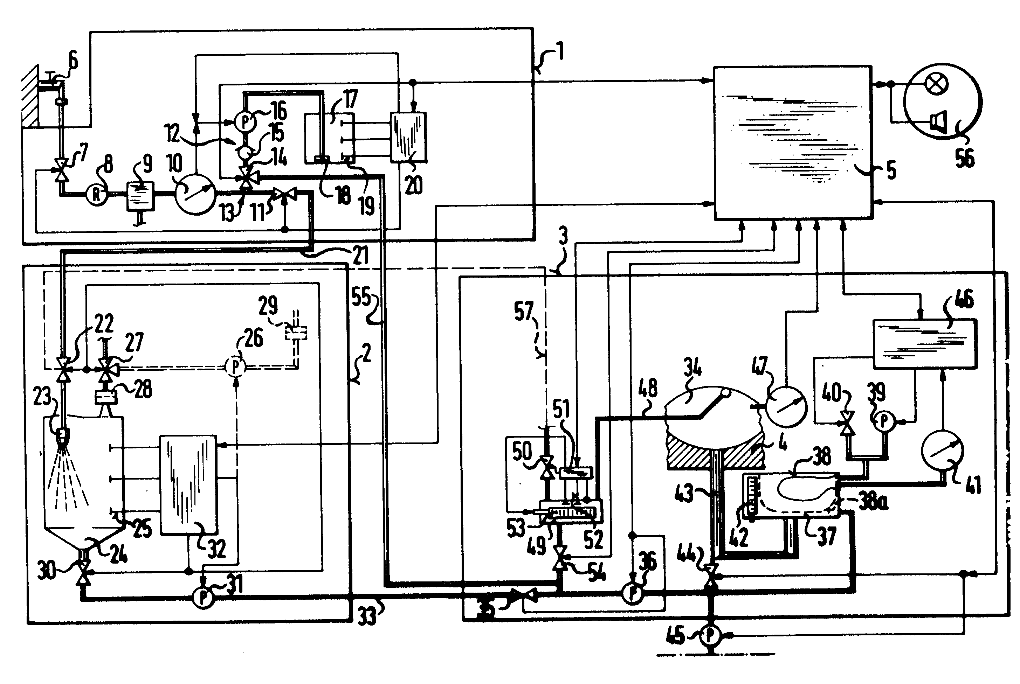

Die Erfindung ist nachstehend anhand eines in der anliegenden Zeichnung dargestellten Ausführungsbeispiels näher erläutert. Die einzige Figur zeigt das Blockschaltbild einer erfindungsgemäß ausgestalteten Lithotripsie-Vorrichtung.

Die erfindungsgemäße Vorrichtung besteht im wesentlichen aus einer Desinfektionseinrichtung 1, einer nach dem Prinzip der Vakuumentgasung arbeitenden Wasserentgasungeinrichtung 2, einem Hauptkreislauf 3 zum Befüllen und Entleeren eines Koppelkissens, eines Stoßwellenerzeuges 4 sowie einer zentralen elektronischen Steuereinheit 5 zur Steuerung der Einrichtungen 1, 2 und 3.

Die Desinfektionseinrichtung 1 dient der Verhinderung des Wachstum und der Reduzierung von Bakterien, Viren, Pilzen, Algen und ähnlichem, um eine Verschleimung des Wassers zu verhindern sowie um eine mögliche Kontamination von Patienten auszuschließen, und ist vorzugsweise über einen Wasserzulauf 6 an das Trinkwassernetz anschließbar. Der Anschluß erfolgt über ein erstes Magnetventil 7, dem ein Durchflußregler 8, ein Rohrtrenner 9, eine Volumenstrommeßeinrichtung 10 und ein zweites Magnetventil 11 in Reihe nachgeschaltet sind. An die Verbindungsleitung zwischen dem Magnetventil 11 und der Volumenstrommeßeinrichtung 10 ist eine Dosiereinrichtung 12 angeschlossen, die, von der als Impfstelle 13 fungierenden Anschlußstelle aus betrachtet, in Reihe geschaltet ein drittes Magnetventil 14, ein Rückschlagventil 15, eine Dosierpumpe 16 und einen Vorratsbehälter 17 mit Absaugrohr 18 und Niveaufühlern 19 unfaßt. Die Steuerung der Desinfektionseinrichtung 1 erfolgt über eine elektrische Steuerschaltung 20.

Die Desinfektionseinrichtung 1 ist über eine Rohrleitung 21, die an dem Ausgang des zweiten Magnetventils 11 angeschlossen ist, mit der Wasserentgasungseinrichtung 2 verbunden. Der Anschluß erfolgt an einem Magnetventil 22, das eine Einspritzdüse 23 mit Wasser beschickt, die in einen Entgasungsbehälter 24 mit Niveaufühlern 25 mündet. An den Entgasungsbehälter 24 ist eine Vakuumpumpe 26 angeschlossen, die über ein Magnetventil 27 und einen wasserdichten Filter 28 mit dem Innern des Entgasungsbehälters 24 verbunden ist. Der Vakuumpumpe 26 kann ein Filter 29 nachgeschaltet sein, über den das abgesaugte Gas in die Atmosphäre entlassen wird. Der Ablauf des Entgasungsbehälters 24 führt über ein Magnetventil 30, dem eine Pumpe 31 nachgeschaltet ist. Die Steuerung der Wasserentgasungseinrichtung 2 erfolgt über ehe Steuerschaltung 32.

Die Pumpe 31 ist ausgangsseitig über eine Rohrleitung 33 mit dem Hauptkreislauf 3 zum Befüllen und Entleeren des Koppelkissens 34 des Stoßwellenerzeugers 4 verbunden. Dazu mündet die Rohrleitung 33 in ein Magnetventil 35, das einer Umwälzpumpe 36 vorgeschaltet ist, die ausgangsseitig mit einem Druckausgleichsbehälter 37 in Verbindung steht. Dieser ist mit einem seitlich eingeführten Ballon 38 versehen, der durch eine Pumpe 39 mit Druckluft beaufschlagbar und mit Hilfe eines Magnetventils 40 entleerbar ist. Ein Grenzwertgeber 41 überwacht die Druckverhältnisse in dem Ballon 38. Anstelle du Ballons 38 kann auch eine Membran 38a Verwendung finden, die einen mit Druckluft füllbaren Teilraum in dem Druckausgleichsbehälter 37 abtrennt. In letzterem kann eine Heizung 42 untergebracht sein. Der flüssigkeitsseitige Teil des Druckausgeichsbehälters 37 ist mit dem Koppelkissen 34 des Stoßwellengenerators 4 durch eine Rohrleitung 43 relativ große Querschnitts verbunden. An deren tiefstem Punkt ist ein Magnetventil 44 vorgeshen, das eine Entleerung des Systems über eine Absaugpumpe 45 ermöglicht. Die Steuerung des Druckes in dem Koppelkissen 34 besorgt eine Druckregelung 46.

Der Füllzustand des Flüssigkeitsraumes des Koppelkissens 34 wird durch einen Druckmesser 47 überwacht. Gleichzeitig mündet im Bereich der höchstgelegenen Stelle des Koppelkissens 34 eine Absaugleitung 48, die in einen Behälter 49 führt. Dieser Behälter 49 ist durch ein Ventil 50 entlüftbar, das durch eine Steuerung 51 gesteuert wird. Der Füllzustand des Behälters 49 wird mit Hilfe von Niveaufühlern 52 kontrolliert und sein Inhalt kann durch eine innerhalb angeordnete Heizung 53 aufgeheizt werden. Am Boden des Behälters 49 befindet sich ehe Rohrverbindung die diesen über ein Magnetventil 54 mit der Rohrverbindung zwischen dem Magnetventil 35 und der Umwälzpumpe 36 verbindet. Von dieser Verbindungsstelle führt eine Rohrleitung 55 zu dem Magnetventil 14 in der Desinfektionseinrichtung 1.

Die beschriebene Einrichtung arbeitet wie folgt:
Die an das Trinkwassernetz angeschlossene Einrichtung wird durch Öffnen der Magnetventile 7 und 11 gefüllt. Dabei fließt ein praktisch konstanter Wasserstrom durch den Durchflußregler 8, den Rohrtrenner 9 und da Volumenstrommeßgerät 10 über das Magnetventil 11 in die Zuleitung 21 der Wasserentgasungseinrichtung 2. Dabei wird dem Wasser an der Impfstelle 13 durch die Dosierpumpe 16 Desinfektionsmittel aus dem Vorratsbehälter 17 zugesetzt. Die Dosierpumpe 16 wird über die Volumemstrommeßeinrichtung 10 und die Steuerung 20 angesteuert, wodurch gewährleistet ist, daß das Desinfektionsmittel stets unabhängig vom Druck des Leitungsnetzes entsprechend der tatsächlichen Wassermenge dosiert wird. Bei sehr stark konzentriertem Desinfektionsmittel im Vorratsbehälter 17 wird die Dosierpumpe 16 von der Volumenstrommeßeinrichtung 10 nur intermittierend angesteuert und in den Ansteuerpausen wird durch das Rückschlagventil 15 sichergestellt, daß die Leitung zum Vorratsbehälter gesperrt ist.

Durch die Niveaufühler 19 wird der Füllzustand des Vorratsbehälter 17 für das Desinfektionsmittel bestimmt und der Steuerung 20 gemeldet. Diese sorgt dann dafür, daß bei entleertem Vorratsbehälter 17 die Dosierpumpe 16 nicht mehr von der Volumenstrommeßeinrichtung 10 in Betrieb geommen werden kann und auch dafür, daß die Magnetventile 7 und 11 geschlossen werden bzw., wenn der Vorratsbehälter schon bei Inbetriebnahme der Anlage leer ist, die Magnetventile 7 und 11 erst gar nicht geöffnet werden. Gleichzeitig meldet die Steuerung 20 dies an die zentrale Steuereinheit 5, die dafür sorgt, daß dieser Betriebszustand z.B., optisch und/oder akustisch, durch Meldemittel 56, die z.B. im Bedienpult des Lithotriptors angeordnet sein können, angezeigt wird.

Durch das Magnetventil 11 wird außerdem gewährleistet, daß bei einem evtl. auftretenden Unterdruck in der Leitung 21 der Vorratsbehälter 17 nicht vom nachfolgenden System leergesaugt werden kann. Sollte der eingangsseitige Druck unter einen festgelegten Mindestdruck abfallen, so trennt der Rohrtrenner 9 die Desinfektionseinrichtung 1 vom Trinkwassernetz ab, um zu verhindern, daß Desinfektionsmittel in das Trinkwasernetz gelangen kann.

Über die Rohrleitung 21 strömt das Wasser der Wasserentgasungseinrichtung 2 zu. Dabei wird der Entgasungsbehälter 24 mit der Vakuumpumpe 26 auf einen von der Wassertemperatur abhängigen Druckwert evakuiert. Dieser Solldruck kann mit Hilfe eines Druckaufnehmers oder über die entsprechend der Förderleistung der Vakuumpumpe 26 zu wählende Pumpzeit eingestellt werden. Nach dem Erreichen dieses Druckes wird das Magnetventil 22 vor dem Entgasungsbehälter 24 geöffnet und somit der Weg für das Wasser aus der Desinfektionseinrichtung 1 freigegeben. Das Wasser wird hierbei mit dem in der Desinfektionseinrichtung 1 herrschenden Druck unterstützt durch das Vakuum im Entgasungsbehälter 24, durch die Einspritzdüse 23 in den Entgasungsbehälter gedrückt und gesaugt. Beim Eintreten der Wassertröpfchen in den Entgasungsbehälter 24 werden, infolge von Kavitation, Gasbläschen aus dem Wasser ausgeschieden. Das entstandene Gas wird dann mit der Vakuumpumpe 26 durch den wasserundurchläßigen Filter 28 und das Ventil 27 abgesaugt und über den Filter 29 in die Atmosphäre abgelassen. Hat der Wasserstand im Entgasungsbehälter 24 den benötigten, von den Niveaufühlern 25 gemessenen Füllstand erreicht, so wird der Füllvorgang beendet, indem die Steuerung 32 das Magnetventil 22 schießt.

Durch Einhalten einer gewissen Nachevakuierzeit, d.h. das Vakuum wird nach Beenden des Füllvorgangs noch einige Zeit gehalten, kann erreicht werden, daß Gasbläschen, die aufgrund der hohen Einspritzgeschwindigkeit vom Wasser mitgerissen wurden und nun im Wasser eingemischt sind, aufsteigen können und dann in der beschriebenen Weist abgesaugt werden können. Das dann in der beschriebenen Weise abgesaugt werden können. Das im Entgasungsbehälter 24 nun enthaltene, sehr gut entgaste Wasser kann nach dem Belüften des Entgasungsbehälters über das Ventil 27 und den wasserdichten Filter 28 sodann über das Magnetventil 30 der Einrichtung 3 zum Füllen des Stoßwellengenerators 4 zugeführt werden. Anschließend kann in der beschriebenen Art weiteres Wasser entgast und der Fülleinrichtung 3 zugeführt werden. Dieser Entgasungs- und Füllzyklus wird solange fortgeführt, bis der Stoßwellenerzeuger 4 mit der nötigen Menge an entgastem Wasser gefüllt ist.

Nach einer anderen vorteilhaften Ausgestaltung der Entgasungseinrichtung 2 kann auf eine teure Vakuumpumpe 26 verzichtet werden, wenn zwischen dem Magnetventil 30 der Entgasugseinrichtung 2 und der Zuführleitung 33 zum Stoßwellengenerator 4 eine Pumpe 31 eingesetzt wird, die auf der Ansaugseite einen genügenden Unterdruck erzeugen kann, beispielsweise eine Zahnradpumpe. Bei einer so ausgeführten Entgasungseinrichtung 2 wird zunächst der nicht evakuierte Entgasungsbehälter 24 über das von der Steuerung 32 angesteuerte Magnetventil 22 mit einer bestimmten Wassermenge aus der Desinfektionseinrichtung 1 gefüllt. Der Füllstand wird dabei wieder von der Steuerung 32 über die Niveaufühler 25 und das Magnetventil 22 gesteuert. Ist der gewählte Füllstand erreicht, so veranlaßt die Steuerung 32, daß das Magnetventil 22 geschlossen, das Magnetventil 30 geöffnet und die Pumpe 31 in Betrieb gesetzt wird. Durch das Absaugen eines Teiles des Wassers aus dem Entgasungsbehälter 24 wird im Entgasungsbehälter in dem Raum zwischen Magnetventil 22 und der Wasseroberfläche ein Vakuum gebildet. Ist der gewünschte Druck im Entgasungsbehälter 24 erreicht, dies kann z.B. auch wieder über die Niveaufühler 25 festgestellt und an die Steuerung 32 weitergemeldet werden so veranlaßt die Steuerung 32 daß das Magnetventil 22 geöffnet wird. Das dann über die Einspritzdüse 23 eintretende Wasser wird nach dem oben beschriebenen Prinzip entgast und die Gasblasen können bei Bedarf über das wasserdichte Filter 28 und das Magnetventil 27 in die Umgebung abgelassen werden. Somit kann ein im Gegensatz zum Entgasungsvorgang mit einer Vakuumpumpe kontinuierlicher Entgasungs- und Füllzyklus erreicht werden.

Das so entgaste Wasser hat einen fast so hohen Entgasungsgrad wie das nach dem ersten Ausführungsbeispiel entgaste Wasser, obwohl es aus entgastem und dem anfangs eingefüllten unentgasten Wasser gemischt ist. Es wird ein Gesamtentgasungsgrad erreicht, da da Volumen an unentgastem Wasser im Verhältnis zum zur Füllung des Stoßwellengenerators 4 benötigten Volumen und damit zur Menge an entgastem Wasser sehr gering ist.

Da entgaste Wasser wird nun der Füll- und Druckregeleinrichtung 3 über die Leitung 33 zugeführt. Das bei geöffnetem Magnetventil 30 und eingeschalteter Pumpe 31 über die Leitung 33 zur Füll- und Druckregeleinrichtung 3 gepumpte Wasser wird nach dem Öffnen des Ventil 35 und Einschalten der Umwälzpumpe 36 von dieser über den Druckausgleichsbehälter 37 in den Stoßwellengenerator 4 gepumpt, bis dieser mit entgastem Wasser vollständig gefüllt ist. Während des Füllvorgangs wird da freie Volumen im Druckausgleichsbehälter 37 für das durchfließende Wasser auf ein Minimum begrenzt. Hierzu wird der seitlich in das Druckausgleichsgefäß eingeführte Ballon 38 durch die Pumpe 39 soweit aufgeblasen, daß er fast das gesamte Volumen des Druckausgleichsbehälter 37 einnimmt, da ja der Stoßwellengenerator 4 und nicht der Ausgleichsbehälter mit Wasser gefüllt werden soll. Der Stoßwellengenerator 4 befindet sich hierzu in der Position, in der ein Maximum an Wasser aufgenommen werden kann.

Die im Stoßwellengenerator 4 und der Verbindungsleitung 43, die mit großem Durchmesser ausgelegt ist, damit Schwingungen der Regelung aufgrund verzögerter Druckänderungserfassung minimal gehalten werden können, vor dem Füllen mit Wasser befindliche Luft wird über die Absaugleitung 48 in den Behälter 43 geleitet Dieser Behälter ist durch da Ventil 50, daß von der Steuerung 51 angesteuert wird, entlüftbar. Dabei bleibt da Ventil 50 solange geöffnet, bis der Behälter 49 und damit such der Stoßwellengenerator 4 vollständig mit Wasser gefüllt sind. Der Füllzustand des Behälters wird mit Hilfe der Niveaufühler 52 ermittelt und entsprechend wird das Ventil 50 von der Steuerung 51 angesteuert.

Der Füllzustand des Stoßwellengenerators 4 wird durch den Druckmesser 47 überwacht, der bei Erreichen eines voreingestellten Druckwertes ein Signal an die zentrale Steuereinheit 5 gibt, worauf diese die Desinfektionseinrichtung 1 und die Entgasungseinrichtung 2 durch entsprechende Steuersignale außer Betrieb setzt, das Magnetventil 35 schließt und das Magnetventil 54 öffnet. Die Pumpe 36 wälzt nun das Wasser in geschlossenem Kreis um. Hierbei heizt die Heizung 42 das Wasser z.B. auf Körpertemperatur auf. Die Heizung 42 kann über die Steuerung 51 gesteuert werden, da nur geheizt werden darf, wenn sich Wasser um die Heizung herum befindet Durch die Unterbringung der Heizung 43 in dem Druckausgleichsgefäß 37 kann der Behälter 49 Sehr klein gehalten werden, die Steuerung 51 mit den Niveaufühlern 52 kann entfallen und das Ventil 50 kann beispielsweise durch einen einfachen Schwimmschalter ersetzt werden.

Das beim Positionieren eines Patienten verdrängte Wasser wird vom Ausgleichsbehälter 37 aufgenommen und der Druck im System durch die Druckregelung 46 mit Hilfe des Magnetventils 40 der Luftpumpe 39 und des Grenzwertgebers 41 konstant gehalten. Insbesondere hat der Grenzwertgeber 41 für den Ballondruck die Aufgabe, dafür zu sorgen, daß der Ballon nicht so stark aufgepumpt wird, daß dieser selbst und der Druckausgleichsbehälter 37 beschädigt oder zum Platzen gebracht werden.

Bei Betrieb des Stoßwellengenerators 4 wird das aufbereitete Wasser im geschlossenem Kreislauf hinter dem Magnetventil 35 zwischen Druckausgleichsbehälter 37 und Stoßwellengenerator 4 durch die Umwälzpumpe 36 umgewälzt und durch die Heizung 42 bzw. 53 auf der gewünschten Temperatur gehalten.

Um das aufbereitete Wasser möglichst lange in der Lithotripsie-Vorrichtung belassen zu können, steuert die zentrale Steuereinrichtung 5 bei Bedarf die Desinfektionseinrichtung 1 so an, daß Desinfektionsmittel über das Magnetventil 14 und die Leitung 55 zur Impfstelle 13 des geschlossenen Wasserkreislaufs gelangt.

Beim Betrieb der Lithotripsie-Vorrichtung eventuell entstehende Gablasen, z.B. durch Kavitation bei der Stoßwellentherapie oder durch Ausgasen des Desinfektionsmittels, werden auf die gleiche Weise wie die beim Füllvorgang vorhandene Luft durch den Absaugschlauch 48 aus dem Koppelkissen 34 entfernt und über den Behälter 49 und das Ventil 50 in die Atmosphäre entlasen.

Eine Entleerung der Anlage ist jederzeit über das Magnetventil 44 und die Absaugpumpe 45 möglich und kann ebenfalls über die zentrale Steuereinheit 5 veranlaßt werden

Es ist natürlich auch möglich, die übrigen Einheiten der genannten Ablage mit einer entsprechenden Vorrichtung zur Entleerung zu versehen.

Schließlich kann für das Absaugen größerer Mengen an Luft und/oder Wasser aus dem Koppelkissen 34, wie es z.B. bei einem Membranwechsel notwendig ist, eine Änderung des Hauptkreislaufes 3 dahingehend erfolgen, daß die erwähnten Bauteile 49, 51, 51 und 53 entfallen Das Ventil 50 ist in diesem Fall durch ein solches mit einem Eingang und mit zwei Ausgängen ersetzt und über eine gestrichelt angedeutete Rohrleitung 57 mit dem Magnetventil 22 der Entgasungseinrichtung 2 verbunden. Das Absaugen erfolgt dann über die Leitung 48 direkt an das Ventil 50 und weiter über die Leitung 57 an da Ventil 22. Die Ventile 50 und 54 sind in diesem Fall nur über eine Rohrleitung miteinander verbunden.

Bei entsprechender Wahl des Desinfektionsmittels, z.B. eines solchen, von dem unter 0,5 ppm zugesetzt werden müssen, kann der Vorratsbehälter 17 der Desinfektionseinrichtung 1 sehr klein gehalten werden und dennoch eine für ein ganzes Wartungsintervall ausreichende Desinfektionsmittelmenge enthalten

## Patentansprüche

1. Lithotripsie-Vorrichtung mit einem Stoßwellengenerator, umfassend ein an den Körper des Patienten anschmiegbares, Wasser als akustisches Koppelmedium zwischen dem Stoßwellengenerator (4) und dem Körper des Patienten aufnehmendes Koppelkissen (34), eine Einrichtung (3) zum Füllen und Entleeren des Koppelkissens (34), eine Einrichtung zur Regelung des Flüssigkeitsdruckes in dem Koppelkissen (34) und eine Einrichtung (2) zum Entgasen des Koppelmediums, dadurch gekennzeichnet, daß vor der Entgasungseinrichtung (2) eine Desinfektionseinrichtung (1) vorgesehen ist, die einen Vorratsbehälter (17) zur Aufnahme eines Desinfektionsmittels, eine Steuerschaltung (20) für den Vorratsbehälter (17) und eine mit dem Vorratsbehälter (17) verbundene Dosiereinrichtung (12) zur Abgabe von Desinfektionsmittel an du Koppelmedium enthält.

2. Vorrichtung nach Anspruch 1, dadurch gekennzeichnet, daß die Dosiereinrichtung (12) aus einer Dosierpumpe (16), einem dieser nachgeordneten Rückschlagventil (15) und aus einem Magnetventil (14) zur Eingabe des abgemessenen Desinfektionsmittels in das Koppelmedium besteht.

3. Vorrichtung nach Anspruch 2, dadurch gekennzeichnet, daß die Dosierpumpe (16) intermittierend betreibbar ist.

4. Vorrichtung nach Anspruch 1, dadurch gekennzeichnet, daß eine Verbindungs-Rohrleitung (55) vorgesehen ist, die die Einrichtung (3) zum Füllen und Entleeren des Koppelkissens (34) mit der Desinfektionseinrichtung (1) verbindet,

5. Vorrichtung nach Anspruch 1, dadurch gekennzeichnet, daß das Koppelkissen (34) mit einem Druckausgleichsgefäß (37) kommuniziert, welches einen mit Gas füllbaren Raumteil umfaßt, der durch eine flexible Haut von dem mit Koppelmedium füllbaren Restraum abgetrennt ist.

## Claims

1. Lithotripsy device having a shock wave generator, comprising a coupling cushion (34) containing water as the acoustic coupling medium between the shock wave generator (4) and the body of the patient and which can be moulded against the body of the patient, a device (3) for filling and emptying the coupling cushion (34), a device for controlling the liquid pressure in the coupling cushion (34) and a device (2) for degassing the coupling medium, characterised in that a disinfecting device (1) containing a supply container (17) for receiving a disinfectant, a control circuit (20) for the supply container (17) and a metering device (12) connected to the supply container (17) and for releasing disinfectant to the coupling medium, is provided upstream of the degassing device (2).

2. Device according to claim 1, characterised in that the metering device (12) comprises a metering pump (16), a return valve (15) arranged downstream of the latter and a solenoid valve (14) for introducing the measured amount of disinfectant into the coupling medium.

3. Device according to claim 2, characterised in that the metering pump (16) can be operated intermittently.

4. Device according to claim 1, characterised in that a connection pipe (55) is provided which connects the device (3) for filling and emptying the coupling cushion (34) with the disinfecting device (1).

5. Device according to claim 1, characterised in that the coupling cushion (34) communicates with a pressure balancing vessel (37), which comprises a compartment which can be filled with gas and which is separated from the remaining space which can be filled with coupling medium by means of a flexible skin.

## Revendications

1. Dispositif de lithotripsie muni d'un générateur d'ondes de choc, englobant un coussin de couplage (34) qui peut épouser la forme du corps du patient, dans lequel vient se loger de l'eau à titre de milieu de couplage acoustique entre le générateur d'ondes de choc (4) et le corps du patient, un mécanisme (33) pour remplir et vider le coussin de couplage (34), un dispositif pour régler la pression de liquide régnant dans le coussin de couplage (34) ainsi qu'un mécanisme (2) pour purger le milieu de couplage, caractérisé en ce que, avant le mécanisme de purge (2), on prévoit un mécanisme de désinfection (1) qui contient un récipient d'alimentation (17) pour recevoir un agent de désinfection, un commutateur de commande (20) pour le récipient d'alimentation (17) et un mécanisme de dosage (12) relié au récipient d'alimentation (17) pour amener l'agent de désinfection au moyen de couplage.

2. Dispositif selon la revendication 1, caractérisé en ce que le mécanisme de dosage (12) est constitué par une pompe de dosage (16), par une soupape anti-retour (15) montée à la suite de cette dernière et par une vanne magnétique (14) pour introduire l'agent de désinfection mesuré dans le milieu de couplage.

3. Dispositif selon la revendication 2, caractérisé en ce que la pompe de dosage (16) peut être mise en service de manière intermittente.

4. Dispositif selon la revendication 1, caractérisé en ce qu'on prévoit un conduit tubulaire de raccordement (55) qui relie le mécanisme (3) pour remplir et vider le coussin de couplage (34) au mécanisme de désinfection (1).

5. Dispositif selon la revendication 1, caractérise en ce que le coussin de couplage (34) communique avec un récipient de compensation de pression (37), qui englobe un espace partiel qui peut être rempli avec du gaz, qui est séparé par une peau flexible de l'espace résiduel apte à être rempli par le milieu de couplage.
